# EUROPEAN PATENT SPECIFICATION

(11) **EP 4 181 850 B1**
(45) Date of publication and mention of the grant of the patent: **28.10.2026**
(21) Application number: 21752476.8
(22) Date of filing: 16.07.2021
(51) Int. Cl.: A61F 13/56, C09J 153/02

(54) **HOT MELT PRESSURE SENSITIVE ADHESIVE COMPOSITIONS AND ARTICLES INCLUDING THE SAME**
HEISSSCHMELZ-HAFTKLEBSTOFFZUSAMMENSETZUNGEN UND GEGENSTÄNDE, DIE DIESE ENTHALTEN
COMPOSITIONS D`ADHÉSIF THERMOFUSIBLE SENSIBLE À LA PRESSION ET ARTICLES LES COMPRENANT

(30) Priority: 17.07.2020 US 202063053057 P
(43) Date of publication of application: 24.05.2023
(73) Proprietor: H.B. Fuller Company, St. Paul, Minnesota 55164-0683 (US)
(72) Inventor: EVERSON, Daniel A., Lakeville, MN 55044 (US); MICHEL, Andrew R., Plymouth, MN 55446 (US); MANSOUR, Ameara S., Woodbury, MN 55129 (US)
(74) Representative: Maiwald GmbH
(86) International application number: PCT/US2021/070887
(87) International publication number: WO 2022/016193

(56) References cited:
- EP-A1- 0 525 251
- EP-A1- 1 564 273
- EP-B1- 1 025 178
- WO-A1-2015/109160
- US-A1- 2005 181 207
- US-B1- 6 197 845
- US-B1- 7 442 739
- ANONYMOUS: "Eastotac(TM) H-142R Resin | TDS | Eastman Chemical Company", 21 April 2021 (2021-04-21), pages 1 - 3, XP055852922, Retrieved from the Internet <URL:https://productcatalog.eastman.com/tds/ProdDatasheet.aspx?product=71000807&pn=Eastotac+H-142R+Resin#_ga=2.118737225.132993917.1634627270-1678663842.1634627270> [retrieved on 20211019]
- KIM DAE-JUN ET AL: "Tack and fracture energy of tackified SIS (styrene-isoprene-styrene)-based hot-melt pressure sensitive adhesives (HMPSAs)", JOURNAL OF ADHESION SCIENCE AND TECHNOLOGY, vol. 20, no. 12, 1 January 2006 (2006-01-01), GB, pages 1367 - 1381, XP055852924, ISSN: 0169-4243, DOI: 10.1163/156856106778456618
- TOTAL: "Wingtack 95", 1 October 2019 (2019-10-01), pages 1 - 1, XP055852925, Retrieved from the Internet <URL:https://www.crayvalley.com/docs/TDS/wingtack-95-flake.pdf> [retrieved on 20211019]

## Description

### BACKGROUND

This application is directed to improved hot melt adhesive compositions for positioning.

Positioning adhesives are pressure sensitive adhesives, commonly hot melt pressure sensitive adhesives, used on disposable absorbent (e.g. feminine hygiene) articles to position the article on a substrate (e.g. undergarment).

In recent years, there has been an increased interest in formulating hot melt pressure sensitive adhesives to eliminate compounds that can be found in raw materials that could potentially be used to formulate hot melt adhesives and may be perceived by consumers as unsafe. These compounds are sometimes referred to as substances of interest (SOI) and include materials such as primary dioxin (e.g. octachlorodibenzofuran) and dioxin like polychlorinated biphenyls.

The variety of garments and detergents used in the world is also increasing, and these variables have an impact on bonding performance. Two common fabrics used today are cotton and microfiber.

In addition, to being low in SOI, there is also need for a hot melt pressure sensitive adhesive compositions that exhibits a strong, consistent peel to both cotton and microfiber, yet exhibit little to no adhesive transfer to an undergarment.

WO2015/109160A1 discloses a hot melt positioning adhesive.

### SUMMARY

In one aspect, the invention features an absorbent article according to claim 1 including a topsheet, a backsheet, optionally an absorbent core disposed between the topsheet and the backsheet, a garment facing surface and a hot melt pressure-sensitive adhesive composition disposed on the garment-facing surface of the absorbent article, the hot melt pressure sensitive adhesive composition including from 10% by weight to 35% by weight of a styrenic block copolymer having an average styrene content of from 10% by weight to 22% by weight and an average di-block content of from 12% by weight to 65% by weight, from 30% by weight to 65% by weight of a hydrogenated hydrocarbon tackifying agent having a weight average molecular weight (Mw) of at least 1000 grams/mole and from 10% by weight to 40% by weight of a plasticizer.

In a different embodiment, the hydrogenated hydrocarbon tackifying agent has an average R&B softening point of from 95°C to 120°C. In another embodiment, the hydrogenated hydrocarbon tackifying agent has aromatic content. In one embodiment, the hydrogenated hydrocarbon resin is selected from the group consisting of aromatic modified hydrocarbons resin, aromatic hydrocarbon resin and combinations thereof. In a different embodiment, the hydrogenated hydrocarbon resin comprises an aromatic modified hydrocarbon resin and an aromatic hydrocarbon resin.

In one embodiment, the styrenic block copolymer is selected from a group consisting of poly(styrene-b-isoprene-b-styrene), poly(styrene-b-(ethylene-co-butylene)-b-styrene) and combinations thereof. In a second embodiment, the styrenic block copolymer has an average melt flow rate according ASTM D 1238 (200°C, 5-kg) of from 5 to 40 grams/10 minutes.

In another embodiment, the styrenic block copolymer has an average diblock content of from 20% by weight to 60% by weight. In one embodiment, the plasticizer is a naphthenic oil.

In a different embodiment, the absorbent article is a feminine hygiene product.

In another aspect, the invention features a hot melt pressure sensitive adhesive composition according to claim 13 including from 12% by weight to 28% by weight of a styrenic block copolymer having an average styrene content of from 10% by weight to 22% by weight and an average di-block content of from 12% by weight to 65% by weight, from 30% by weight to 65% by weight of a hydrogenated hydrocarbon tackifying agent having a weight average molecular weight (Mw) of at least 1000 grams/mole and aromatic content, and from 10% by weight to 40% by weight of a plasticizer.

In one embodiment, hot melt pressure sensitive adhesive is free of dioxins.

In another embodiment, the hot melt pressure sensitive adhesive is free of non-hydrogenated C5 tackifying agents.

In a different embodiment, the hydrogenated hydrocarbon tackifying agent comprises an aromatic modified hydrocarbon tackifying agent having an aromatic content of from 3% to 15% and an aromatic hydrocarbon tackifying agent having an aromatic content of from 40% to 100%.

The hot melt pressure sensitive adhesive (HMPSA) compositions of this invention utilize selected styrene block copolymer and hydrogenated hydrocarbon tackifying agent/s to formulate compositions that are low in SOI, exhibit strong, consistent peel to both cotton and microfiber, and do not result in adhesive transfer.

Other features and advantages will be apparent from the following description of the preferred embodiments and from the claims.

### DETAILED DESCRIPTION

The hot melt pressure sensitive adhesive (HMPSA) composition can include from 10% by weight to 35% by weight of a styrenic block copolymer having an average styrene content of from 10% by weight to 22% by weight and an average di-block content of from 12% by weight to 65% by weight, from 30% by weight to 65% by weight of a hydrogenated hydrocarbon tackifying agent having a weight average molecular weight (Mw) of at least 1000 grams/mole and from 10% by weight to 40% by weight of a plasticizer.

The hot melt pressure sensitive adhesive composition can include from 12% by weight to 28% by weight of a styrenic block copolymer having an average styrene content of from 10% by weight to 22% by weight and an average di-block content of from 12% by weight to 65% by weight, from 30% by weight to 65% by weight of a hydrogenated hydrocarbon tackifying agent having a weight average molecular weight (Mw) of at least 1000 grams/mole and aromatic content, and from 10% by weight to 40% by weight of a plasticizer.

The HMPSA composition is suitable for use as a positioning adhesive, which is a class of adhesive compositions that is often used to position feminine hygiene articles, such as sanitary napkins, on undergarments. These articles are removed from the undergarment after use. When the article is removed from the undergarment, preferably no adhesive composition remains on the undergarment (i.e., the undergarment is free of adhesive transfer).

The HMPSA compositions of this invention have strong, consistent adhesion (as tested by peel) to both cotton and microfiber. Consistent peel across fabrics is important as the user desires the same level of performance of the disposable absorbent article (e.g. feminine hygiene article) regardless of the substrate it is bonded to (e.g. undergarment fabric).

Due to the polarity and the tight weave of microfiber fabric, it can be difficult for generally non-polar hot melt adhesive compositions to have consistent adhesion to both cotton and microfiber. The HMPSA composition can have a peel to microfiber of +/- 25%, +/- 20%, +/- 15%, or even +/- 10% of the peel to cotton when tested according to the Cotton and Microfiber Peel Force Test Method

The HMPSA composition can be free of dioxins. Consumers have a preference for materials denoted as not having SOIs, such as primary dioxin (e.g. octachlorodibenzofuran (OCDF) and dioxin like polychlorinated biphenyls.

The composition also preferably exhibits a viscosity of no greater than 20,000 centipoise (cP), no greater than 15,000 cP, no greater than 10,000 cP, from 100 cP to 20,000 cP, or even from 100 cP to 15,000 cP at 149°C.

### STYRENIC BLOCK COPOLYMER

The composition includes a styrenic block copolymer.

The styrenic block copolymer has at least one A block that includes styrene and at least one B block that includes, e.g., elastomeric conjugated dienes (e.g., hydrogenated and unhydrogenated conjugated dienes), sesquiterpenes (e.g., hydrogenated and nonhydrogenated sesquiterpenes), and combinations thereof. The A blocks and the B blocks bind to one another in any manner of binding such that the resulting copolymer exhibits a variety of structures including, e.g., random, straight-chained, branched, radial, star, comb, tapered, and combinations thereof. The block copolymer can exhibit any form including, e.g., linear A-B block, linear A-B-A block, linear A-(B-A) n-B multi-block, and radial (A-B)n-Y block where Y is a multivalent compound and n is an integer of at least 3, tetrablock copolymer, e.g., A-B-A-B, and pentablock copolymers having a structure of A- B-A-B-A. The adhesive composition can include blends of at least two different block copolymers.

Suitable styrene A blocks include, e.g., styrene, alpha-methylstyrene, o-methylstyrene, m-methylstyrene, p-methylstyrene, p-tert-butylstyrene, 2,4- dimethylstyrene, 2,4,6-trimethylstyrene, and combinations thereof.

Suitable block elastomeric conjugated diene B blocks include, e.g., butadiene (e.g., polybutadiene), isoprene (e.g., polyisoprene), 2,3-dimethyl-1,3-butadiene, 1 ,3-pentadiene, 1,3-hexadiene, and combinations thereof, and hydrogenated versions thereof including, e.g., ethylene, propylene, butylene and combinations thereof. Suitable B block sesquiterpenes include, e.g., beta farnesene. The styrenic block copolymer can have an unsaturated mid-block, alternately the mid-block can be saturated i.e. hydrogenated.

In addition to elastomeric conjugated diene B blocks, styrene monomer can be distributed in the mid-block.

Useful styrenic block copolymers include, e.g., poly(styrene-*b*-butadiene) (SB), poly(styrene-*b*-butadiene-*b*-styrene) (SBS), poly(styrene-*b*-isoprene) (SI), poly(styrene- *b-*isoprene-*b-*styrene) (SIS), poly(styrene-b-(ethylene-*co*-butylene)-*b*-styrene) (SEBS), poly(styrene-*b-*(ethylene-*alt*-propylene)-*b*-styrene) (SEPS), poly(styrene-*b*-isobutylene-*b-*styrene) (SIBS), poly(styrene-*b*-(ethylene-*co*-butylene-*co*-styrene)-*b*-styrene) (SEBSS) and combinations thereof.

Preferred block copolymers include poly(styrene-*b*-isoprene-*b*-styrene) (SIS), poly(styrene-(ethylene-*co*-butylene)-*b*-styrene) (SEBS), poly(styrene-*b*-(ethylene-*alt-*propylene)-*b*-styrene) (SEPS), poly(styrene-*b*-isobutylene-*b*-styrene) (SIBS) and combinations thereof.

Particularly preferred block copolymers include poly(styrene-*b*-isoprene-*b*-styrene) (SIS), poly(styrene-*b*-(ethylene-*co*-butylene)-*b*-styrene) (SEBS) and combinations thereof.

The styrenic block copolymer can include more than one styrenic block copolymer. When more than one styrenic block copolymer is included the styrene content, the diblock content and the melt flow rate ranges specified below are a weight average of the various grades present.

As an example, if the HMPSA composition comprises two styrenic block copolymers A and B. Polymer A is present at 25 weight % (*w_{A}*) with a styrene content of 15% (*s_{A}*) and polymer B is present at 25 weight % (*w_{B}*) with a styrene content of 20 weight % (*s_{B}*). The average styrene content of the styrenic block copolymer is calculated in the following way: ${w}_{A} / \left({w}_{A}+{w}_{B}\right) * {s}_{A} + {w}_{B} / \left({w}_{A}+{w}_{B}\right) * {s}_{B} = 0.5 \left(15\right) + 0.5 \left(20\right) = 17.5 weight % .$

The styrenic block copolymer can have an average styrene content of from from 10% by weight to 22% by weight, or from 10% by weight to 20% by weight.

The styrenic block copolymer can have an average diblock content of from 12% by weight to 65% by weight, from 15% by weight to 60% by weight, or even from 20% by weight to 60% by weight.

The inventors have found that using a styrenic block copolymer with an average styrene content of no greater than no greater than 22% by weight or even no greater than 20% and an average di-block content of from 12% by weight to 65% by weight or even from 20% by weight to 60% by weight results in a HMPSA composition having a good balance of adhesion (as witnessed by the cotton and microfiber peels) and cohesion (as witnessed by lack of transfer).

The styrenic block copolymer can have an average Melt Flow Rate (MFR) per ASTM D 1238 (200°C/5 kg) in g/ 10 min of from 5 to 40, 8 to 35, or even 9 to 30.

Useful block copolymers are commercially available under the KRATON D and KRATON G series of trade designations from Kraton Corporation, from (Houston, Texas) including KRATON D1113, KRATON D1193, KRATON D1124, KRATON D1113, KRATON G1657 and KRATON G-1643, the VECTOR series of trade designations from Taiwan Synthetic Rubber Corporation (TSRC) (Taipei City, Taiwan) including VECTOR 4114, VECTOR 4186, VECTOR 4215, VECTOR 4293, VECTOR 4116, and VECTOR 4113, and the GLOBALPRENE series of trade designations from LCY Grit Corp (Taiwan) including GLOBALPRENE 5516D.

The composition includes from 10% by weight to 35% by weight, from 10% by weight to 30% by weight, or even from 12% by weight to 28% by weight styrenic block copolymer.

### HYDROGENATED HYDROCARBON TACKIFYING AGENT

The HMPSA compositions of this invention can be free of non-hydrogenated tackifying agents (e.g. non-hydrogenated C5 resins). Non-hydrogenated tackifying agents have been found to increase the color, odor, and increase the degradation at high temperature of the HMPSA composition. Hydrogenated hydrocarbon tackifying agents are lower in odor and color and have been shown to have improved heat stability.

The HMPSA composition can include more than one hydrogenated hydrocarbon tackifying agent. When more than one or more hydrogenated hydrocarbon tackifying agent is included the softening point and molecular weight ranges specified below are a weight average of the grades present.

The inventors have found that hydrogenated hydrocarbon tackifying agents within a particular range of both softening point and molecular weight can be helpful in optimizing peel properties.

The hydrogenated hydrocarbon tackifying agent can have an average Ring and Ball softening point of from 90°C to 130°C, 95°C to 120°C, or even 100°C to 115°C. The hydrogenated hydrocarbon tackifying agent can have an average Molecular Weight -Weight Average (Mw) of at least 1000 g/mole, from 1000 g/mole to 2000 g/mole.

The hydrogenated hydrocarbon tackifying agent can have an average aromatic content of from 10% to 25%, or even from 12% to 20%.

Suitable classes of hydrogenated hydrocarbon tackifying agents include, e.g., aromatic, aliphatic and cycloaliphatic hydrocarbon resins, aromatic modified aliphatic or cycloaliphatic hydrocarbon resins, terpenes, modified terpenes and combinations thereof. Examples of useful aliphatic and cycloaliphatic petroleum hydrocarbon resins include aliphatic and cycloaliphatic petroleum hydrocarbon resins include, e.g., branched and unbranched C9 resins and C10 resins. Examples of useful polyterpene resins include polyterpene resins, and copolymers and terpolymers of natural terpenes (e.g. styrene-terpene, alpha-methyl styrene-terpene and vinyl toluene-terpene).

Preferred classes of hydrogenated hydrocarbon tackifying agents include hydrogenated hydrocarbon resins having aromatic content. Hydrogenated hydrocarbon resins having aromatic content can be selected from the group consisting of aromatic modified hydrocarbon resin and aromatic hydrocarbon resin.

The aromatic modified hydrocarbon resins are primarily non-aromatic but include from 3% to 15% of aromatic content. The aromatic content is measured via proton Nuclear Magnetic Resonance (NMR) integration. Aromatic modified hydrocarbon resins can be selected from aliphatic and cycloaliphatic hydrocarbon resins, terpenes and modified terpenes.

The aromatic hydrocarbon resin is derived from aromatic vinyl monomers. Suitable examples of the aromatic hydrocarbon resins include but are not limited to aromatic hydrocarbon resins comprising monomers selected from the group consisting of styrene, alpha methyl styrene, vinyl toluene, indene, or any other aromatic monomer or end block associating monomer. Aromatic hydrocarbon resins can have greater than 40%, greater than 50 %, greater than 60%, from 40% to 100%, or even from 50% by weight to 100% of aromatic content. The aromatic content is measured via proton Nuclear Magnetic Resonance (NMR) integration.

Useful hydrogenated hydrocarbon resins include those available under the ESCOREZ trade designation from ExxonMobil Chemical Company including ESCOREZ 5600 (Molecular Weight -Weight Average (Mw) = 800 g/mole, 9.8% aromatic content), ESCOREZ 5690 (10% aromatic content), ESCOREZ 5615 (9.9% aromatic content), ESCOREZ 5637 (5% aromatic content) and ESCOREZ 5400 (0% aromatic content, Mw = 670 g/mole).

Useful hydrogenated aromatic hydrocarbon resins include KRISTALEX and PLASTOLYN series of trade designations from Eastman Chemical Company (Kingsport, Tenn.) including, e.g., KRISTALEX 3100, PLASTOLYN 240 (Mw = 4060, 55% aromatic content) and PLASTOLYN 290 (Mw =4760).

The HMPSA composition includes from about 30% by weight to 65% by weight, from about 35% by weight to 60 % by weight, or even from 40% by weight to 60% by weight hydrogenated hydrocarbon tackifying agent.

In addition to the above, other useful hydrogenated hydrocarbon tackifying agents are commercially available under a variety of trade designations including, e.g., the EASTOTAC series of trade designations from Eastman Chemical (Kingsport, Tennessee) including, e.g., EASTOTAC H-100R and H-100L.

### PLASTICIZER

The plasticizer can be a liquid at room temperature. Suitable plasticizers for use in the composition include, e.g., naphthenic oil, mineral oil, paraffin oil, synthetic liquid oligomers of polyolefins (e.g., polybutene and polypropylene), hydrocarbon fluids, vegetable oil, and combinations thereof.

Useful commercially available plasticizers include, e.g., plasticizers sold under the NYFLEX series of trade designations from Nynas Corporation (Houston, Texas) including, e.g., NYFLEX 222B and NYFLEX 223, KAYDOL OIL from Sonneborn, LLC (Parsippany, New Jersey), KRYSTOL 550 mineral oil from Petrochem Carless Limited (Surrey, England), and CALSOL 5550 oil from Calumet Specialty Products Partners, LP (Indianapolis, Indiana),

The composition includes from 10% by weight to 40% by weight, from 12% by weight to 35% by weight, or even from 15% by weight to 28% by weight of a plasticizer.

### OPTIONAL COMPONENTS

The composition can optionally include a variety of additional components including, e.g., stabilizers, antioxidants, adhesion promoters, ultraviolet light stabilizers, rheology modifiers, biocides, corrosion inhibitors, dehydrators, colorants (e.g., pigments and dyes), optical brighteners, fillers, surfactants, flame retardants, waxes, additional tackifying agents, additional polymers, and combinations thereof.

Useful antioxidants include, e.g., pentaerythritol tetrakis[3,(3,5-di-tert-butyl-4-hydroxyphenyl)propionate], 2,2'-methylene bis(4-methyl-6-tert-butylphenol), phosphites including, e.g., tris-(p-nonylphenyl)-phosphite (TNPP) and bis(2,4-di-tert-butylphenyl)4,4'-diphenylene-diphosphonite, di-stearyl-3,3'-thiodipropionate (DSTDP), and combinations thereof. Useful antioxidants are commercially available under a variety of trade designations including, e.g., the IRGANOX series of trade designations including, e.g., IRGANOX 1010, IRGANOX 565, and IRGANOX 1076 hindered phenolic antioxidants and IRGAFOS 168 phosphite antioxidant, all of which are available from BASF Corporation (Florham Park, New Jersey), and ETHYL 702 4,4'-methylene bis(2,6-di-tert-butylphenol), and the BNX series of trade designations including, e.g., BNX 1010 and BNX 1076 from Mayzo, Inc. (Suwanee, Georgia). When present, the adhesive composition preferably includes from about 0.1 % by weight to about 2 % by weight antioxidant.

### USE

The hot melt pressure sensitive adhesive composition is suitable for use in a variety of articles including, e.g., absorbent articles (e.g., absorbent hygiene articles), tapes (e.g., repositionable pressure sensitive adhesive tapes), and combinations thereof. Useful absorbent hygiene articles include an absorbent structure and the pressure sensitive adhesive composition disposed on a surface of the absorbent structure. The absorbent hygiene article, which is designed to be used and disposed of after a single use, can have a variety of constructions and can be suitable for use in a variety of applications including, e.g., feminine hygiene pads (e.g., panty liners and sanitary napkins), bed liners, incontinence pads, dress shields, and nursing pads. The absorbent structure of the absorbent hygiene article can include a variety of components arranged in a variety of configurations.

The pressure-sensitive adhesive composition is useful as a positioning adhesive disposed on at least one substrate surface of a disposable absorbent article and can be used to position an absorbent article on a garment such as underwear. Such disposable absorbent articles include, e.g., feminine hygiene articles such as sanitary napkins and panty liners, diapers, disposable garments having a waist opening and leg openings, and adult incontinence articles.

In one construction, the disposable absorbent article (e.g., a feminine hygiene article) includes a garment facing surface and a body facing surface, a topsheet having a garment facing surface and a body facing surface, a backsheet having a garment facing surface and a body facing surface, and optionally an absorbent core disposed between the body facing surface of the backsheet and the garment facing surface of the topsheet.

The absorbent core can be optional as for absorbent articles having limited absorption, a specific top sheet e.g. high lofted top sheet can also serve as the absorbent portion of the article.

The top sheet defines a body-facing surface of the absorbent article. The absorbent core is positioned inwardly from the outer periphery of the absorbent article. The absorbent structure includes a body-facing surface and a garment-facing surface positioned adjacent the backsheet. The positioning adhesive composition is disposed between a release liner and the backsheet, which is permanently adhered to the absorbent core through a permanent adhesive composition. The top sheet is permanently adhered to the absorbent core through a discontinuous permanent adhesive composition. The top sheet and the backsheet are joined together and the sealed edges of the top sheet and the backsheet define an overall sealed peripheral edge of the article. The absorbent article can be of any suitable shape and size.

The top sheet is designed to contact the body of the user and is liquid-permeable. The exposed surface of the liquid permeable top sheet is designed to receive aqueous fluids from the body, which fluids will then be directed away from the body of the user and toward the absorbent core. The top sheet is constructed of any suitable material that is easily penetrated by bodily exudates. The top sheet optionally includes a plurality of apertures formed therethrough to permit body fluid to pass more readily into the absorbent core.

The backsheet is liquid-impermeable and designed to face the inner surface, i.e., the crotch portion of the garment (e.g., underwear) of a user. The backsheet optionally is constructed to permit a passage of air or vapor out of the absorbent article (e.g., a vapor permeable layer), while still blocking the passage of liquids.

The hot melt pressure-sensitive adhesive composition is disposed on the garment facing surface of the adsorbent article, in general, or even on the garment facing surface of the backsheet, in particular. A release liner optionally is disposed on the pressure-sensitive hot melt adhesive composition to protect the pressure-sensitive adhesive composition until use. The absorbent article (e.g., a feminine hygiene article) optionally includes additional layers and adhesives and the components of the absorbent article optionally exhibit additional functionality. Examples of additional layers, functionality and combinations thereof include dusting, wicking, acquisition, additional top sheets, multiple core layers, superabsorbent particles and compositions, wetness indicators, and combinations thereof.

Two common fabrics used today, that positioning adhesives need to bond to, are cotton and microfiber. Cotton fabric refers to a woven fabric made with 100% cotton fiber. Microfiber is a fabric created with synthetic fibers having a diameter of less than ten micrometers that are tightly woven. Multiple chemistries (often polar materials e.g. polyester, polyamides, etc.) can be used to create the microfiber fabrics.

The composition is also useful in a variety of other applications and constructions including, e.g., forming permanent bonds, temporary bonds (e.g., removable and repositionable adhesive applications), medical dressings (e.g., wound care products), bandages, surgical pads, drapes, gowns, labels (e.g., pressure-sensitive adhesive labels), tapes (e.g., pressure-sensitive adhesive tapes), filters (e.g., pleated filters and filter frames), and combinations thereof.

The composition is useful in a variety of forms including, e.g., as a coating (e.g., continuous and discontinuous (e.g., random, pattern, array, spiral, dots, spots, and combinations thereof) coatings), film (e.g., continuous films and discontinuous films), bead, sheet, fiber, filament, web (e.g., woven and nonwoven), and combinations thereof.

The composition also can be applied to a variety of substrates including, e.g., films (e.g., polyolefin (e.g., polyethylene and polypropylene), polyester, metallized polymer, multilayer, biaxially oriented, monoaxially oriented, ethylene-vinyl acetate copolymer, polyurethane, vinyl, polyvinylidene fluoride, cellulose acetate and ethyl cellulose, and polyamide films, and combinations thereof), metal foils, release liners, porous substrates, cellulose substrates, sheets (e.g., paper and fiber sheets), paper products, woven and nonwoven webs, fibers (e.g., natural cellulose fibers such as wood pulp, cotton, silk and wool; cellulosic fibers; synthetic polymer fibers such as nylon, rayon, polyesters, acrylics, polypropylenes, polyethylene, polyvinyl chloride, and polyurethane; glass fibers; recycled fibers; and various combinations thereof), and tape backings. Useful substrates include, e.g., single layer, multilayer, treated (e.g., corona treated or chemically primed), and untreated substrates, and combinations thereof.

Various application techniques can be used to apply the adhesive composition to a substrate including, e.g., slot coating, spraying (e.g., spiral spraying and random spraying), screen printing, foaming, engraved roller, extrusion, meltblown adhesive application techniques, and combinations thereof.

The invention will now be described by way of the following examples. All parts, ratios, percentages and amounts stated in the Examples are by weight unless otherwise specified.

### EXAMPLES

### Test Procedures

Test procedures include the following. All ratios and percentages are by weight unless otherwise indicated. The procedures are conducted at room temperature (i.e., an ambient temperature of from about 20 °C to about 25 °C) unless otherwise specified. The properties set forth for the components used in the compositions are as reported by the manufacturer unless otherwise specified.

### Tackifying Agent Molecular Weight

Tackifying agent molecular weight can be determined by size-exclusion chromatography (SEC) in tetrahydrofuran (THF)
Approximately 0.03 g of each tackifying agent can be mixed with 10 mL THF on a shaker for several hours. A clear solution is obtained which is filtered through a 0.45 µm polytetrafluoroethylene (PTFE) filter.

SEC can be performed with THF as the eluent with two MESOPORE columns at 40 °C, analyzed by an ultraviolet (UV) detector (at 254 nm and 300 nm) then a refractive index (RI) detector at 40 °C, and calibrated to polystyrene standards (molecular weight = 0.371¬-19.50 kDa). The elution time from the UV trace can be adjusted to overlay the RI/UV data (i.e. because the detectors are connected in series, the sample passes through the UV detector 0.2 min before it is detected by RI. To overlay the UV and RI traces for comparison, ~ 0.2 min was added to the elution time of the UV trace.).

Molecular Weight (Mw) can then be calculated and reported out.

### Viscosity Test Method

Viscosity is determined in accordance with ASTM D-3236 entitled, "Standard Test Method for Apparent viscosity of Hot Melt Adhesives and Coating Materials," (October 31, 1988), using a Brookfield Thermosel viscometer Model RVDV 2, and a number 27 spindle. The results are reported in centipoise (cP) and the test is performed at the specified temperature.

### Test Sample Preparation Method for Peel Force to Cotton and Microfiber

A laminate is prepared by coating a sample composition onto a silicone coated release paper in a one inch wide pattern at an add-on weight of 20 grams per square meter (g/m²) (+/-3 g/m²) using a slot applicator and then contacting the adhesive strip with the treated side of a 1 mil (0.025 mm) thick polyethylene film to form a silicone coated release paper/adhesive/polyethylene film laminate. Test samples having a length of 4 inches (in) (10.16 cm) in the machine direction and 1.5 in (3.81 cm) in the cross-machine direction are then cut from the laminate such that the adhesive pattern is centered in the cross-machine direction of the test sample.

For cotton bonds a sheet of 124 g/m² bleached t-shirt cotton fabric (Testfabrics, Inc., West Pittston, Pennsylvania) is cut into strips having a length of 4 in (10.16 cm) in the machine direction and a width of 1.5 in (3.81 cm) in the cross-machine, before cutting the cotton fabric, the grid work of the stitching of the fabric is examined. When the cotton fabric is stretched, the sample will exhibit greater elongation in one direction than in another direction. The cotton fabric is cut lengthwise in the direction that has less elongation. All cotton fabric strips are cut as straight as possible along the stitching grid work. If the cotton fabric strips are cut askew, an inconsistent elongation of the cotton fabric test sample will result.

The release film is removed from the adhesive and the adhesive side of each test sample is gently placed on the surface of a cotton strip such that the cotton curls up (in the lengthwise direction) toward the adhesive bond to form the composite test sample. In preparing the composite test sample, the adhesive is not pressed down onto the cotton fabric.

For the microfiber bonds a sheet of LUCERO polymeric microfiber made from 70 % polyamide and 30 % elastomer having a target weight of 135 g/m2 (Termileniao S.A.). Before testing the fabric is first washed without detergent and then cut into 2 inch by inch strips with the long edge going along the lines of the fabric to avoid stretching.

Care is taken to cut each 8-inch strip in half creating two 4-inch-long strips. Then one of these strips is turned over to reveal the other face of the fabric. An adhesive sample is placed on each of the 4 inch strips ensuring an equal number of bonded samples are made to each face of the fabric.

### Cotton and Microfiber Peel Force Test Method

At least five samples are prepared according to the Peel Force Sample Preparation Method. The test sample is placed on a 2-kg mechanical roll-down device and the roller is allowed to pass over the film side of the sample two times, once in the forward direction and once in the backward direction, at a rate of 305 mm/min. A timer is then activated and the sample is placed into the jaws of INSTRON-type peel tester. The polyethylene film is placed into the moving jaw, and the fabric is attached to the stationary jaw. Within one minute after the sample has been removed from the roll-down device, the sample is tested according to ASTM D1876-01 entitled, "Test Method for Determining Peel Resistance of Adhesive (T-Peel Test Method)," with the exception that the test is run at a rate of 305 mm/min, instead of 250 mm/min, over a period of ten seconds, and at least five replicates are run instead of the ten specified in ASTM D1876. The average peel force over ten seconds of peeling is recorded, and the results are reported in grams.

The peel force is measured 24 hours after the test sample is prepared.

### Transfer

When the cotton and microfiber (or fabric) peel bonds were pulled, the surface of the fabric was evaluated for any HMPSA composition that had transferred. If transfer was seen, a yes was recorded. If no transfer was seen, a no was recorded.

### Examples

The adhesive compositions were prepared by combining and mixing the components under nitrogen in the amounts set forth in Tables 1 in a sigma blade mixer operating at 177 °C.

The adhesive compositions were then tested according to the Viscosity, Peel Force to Cotton, Peel Force to Microfiber and Adhesive Transfer test methods set forth above. The viscosity test method was conducted at 149 °C. These results are also reported in Table 1 below. Example 4 in Table 1 is not according to the invention.

**Table 1**

| | Control 1 | Control 2 | Control 3 | Control 4 | Ex 1 | Ex 2 | Ex 3 | Ex 4 |
|---|---|---|---|---|---|---|---|---|
| VECTOR 4114 (SIS, 15% styrene, 42% diblock, MFR = 25 (200°C. 5kg)) | | | | | 24.5 | | | 22 |
| VECTOR 4186 (SIS, radial, 18% styrene, 73% diblock, MFR= 22 (200°C, 5kg)) | | | | 7 | | 3 | 7.5 | |
| KRATON G1657 (SEBS, 13% styrene, 30% diblock, MFR = 22 (230°C, 5kg)) | | | | | | 12 | 11 | |
| HAI SIS JH 8291 (SIS, <1% diblock, 30% styrene, MFR = 10 (200°C, 5kg)) | 19 | 15 | 12 | 16 | | | | 4 |
| KRATON D11 16 (radial SBS, <1% diblock, 23% styrene,) | | | 7 | | | | | |
| KRATON D 1184 (SBS, < 1% diblock, 30% styrene, MFR=1 (200°C, 5kg) | | 4 | | | | | | |
| HAITACK JH3200 (C5 tackifying agent from Ningbo Jinhai Chenguang Chemical Co., Mw=1747) | 50 | | | | | | | |
| ESCOREZ 5400 | | | 55.5 | | | | | 45.1 |
| ESCOREZ 5600 | | 60.75 | 5 | 56 | 43.5 | 49.5 | 52 | |
| PLASTOLYN 240 | | | | 5 | 8 | 7 | 5 | |
| PLASTOLYN 290 | | | | | | | | 3.5 |
| NYFLEX 223 | 30 | 19 | | 13 | 22 | 26.5 | | 22.5 |
| PURETOL 35 | | | | | | | 22.5 | |
| EPOLENE C-13 | | 0.25 | 0.5 | 2 | 1.1 | 1 | 1 | 2 |
| ADDITIVES | 1.0 | 1 | 1 | 1 | 0.9 | 1 | 1 | 0.9 |
| SBC Average Styrene Content (weight %) | 30 | 30 | 27 | 26.4 | 15 | 14 | 15 | 17 |
| SBC Average Diblock Content (weight %) | < 1 | <1 | <1 | 39 | 42 | 39 | 47 | 36 |
| SBC Average MFR ASTM D-1238 - 200°C, 5-kg (grams/10 minutes) | 10 | 8 | NA | 13.7* | 25 | 22 | 22 | 22.7 |
| Tackifier Average Mw (grams/mole) | 1747 | 800 | 681 | 1061 | 1305 | 1200 | 1087 | 964 |
| Tackifier Average Softening Point (°C) | 99 | 102.5 | 103.3 | 103.8 | 105.2 | 104.7 | 104 | 106 |
| Tackifier Average Aromatic Content (% of aromatic protons) | 3.2 | 9.8 | 1.9 | 13.4 | 16.8 | 15.4 | 13.8 | 5.1 |
| Viscosity @ 149°C (cP) | 1612 | NT | NT | 9650 | 10834 | 4253 | 5800 | 1393 |
| Peel to Cotton (gf/in) | 46 | 210 | 289 | 174 | 189 | 163 | 201 | 166 |
| Peel to Microfiber (gf/in) | 20 | 106 | 200 | 125 | 179 | 149 | 172 | 132 |
| %-Change in Peel | -56.5% | -49.5% | -30.8% | -28.1% | -5.3% | -8.6% | -14.4% | -20.5% |
| Transfer? | Yes | No | No | No | No | No | No | No |

| | | | | | | | | |
|---|---|---|---|---|---|---|---|---|
| *MFR for Kraton G1657 is tested at a higher temperature (230°C versus 200°C) NA (Not Available), NT (Not Tested) | | | | | | | | |

Other embodiments are within the claims.

## Claims

1. An absorbent article comprising:
a) a topsheet;
b) a backsheet;
c) optionally an absorbent core disposed between the topsheet and the backsheet;
d) a garment-facing surface; and
e) a hot melt pressure-sensitive adhesive composition disposed on the garment-facing surface of the absorbent article, the hot melt pressure sensitive adhesive composition comprising:
i) from 10% by weight to 35% by weight of one or more styrenic block copolymer(s), wherein the styrenic block copolymer has a styrene content of from 10% by weight to 22% by weight and a di-block content of from 12% by weight to 65% by weight, and when more than one styrenic block copolymer is included, the styrenic block copolymers have a weight average styrene content of from 10% by weight to 22% by weight and a weight average di-block content of from 12% by weight to 65% by weight,
ii) from 30% by weight to 65% by weight of one or more hydrogenated hydrocarbon tackifying agent(s), wherein the hydrogenated hydrocarbon tackifying agent has a weight average molecular weight (Mw) of at least 1000 grams/mole and, when more than one hydrogenated hydrocarbon tackifying agent is included, the hydrogenated hydrocarbon tackifying agents have a weight average of the weight average molecular weight (Mw) of at least 1000 grams/mole, and
iii) from 10% by weight to 40% by weight of a plasticizer.

2. The absorbent article of claim 1 comprising an absorbent core.

3. The absorbent article of claim 1 wherein the hot melt pressure sensitive adhesive composition is free of dioxins.

4. The absorbent article of claim 1 wherein the hot melt pressure sensitive adhesive composition is free of non-hydrogenated C5 tackifying agents.

5. The absorbent article of claim 1 wherein the hydrogenated hydrocarbon tackifying agent(s) has/have an average Ring and Ball softening point of from 95°C to 120°C.

6. The absorbent article of claim 1 wherein the hydrogenated hydrocarbon tackifying agent(s) has/have an average aromatic content of from 10% by weight to 25% by weight.

7. The absorbent article of claim 1 wherein the hydrogenated hydrocarbon resin is selected from the group consisting of aromatic modified hydrocarbons resin, aromatic hydrocarbon resin and combinations thereof.

8. The absorbent article of claim 1 wherein the hydrogenated hydrocarbon resin comprises an aromatic modified hydrocarbon resin and an aromatic hydrocarbon resin.

9. The absorbent article of claim 1 wherein the styrenic block copolymer is selected from a group consisting of poly(styrene-b-isoprene-b-styrene), poly(styrene-b-(ethylene-co-butylene)-b-styrene) and combinations thereof.

10. The absorbent article of claim 1 wherein the styrenic block copolymer(s) has/have a weight average melt flow rate according ASTM D 1238 (200°C, 5-kg) of from 5 to 40 grams/10 minutes.

11. The absorbent article of claim 1 wherein the styrenic block copolymer(s) has/have an average diblock content of from 20% by weight to 60% by weight.

12. The absorbent article of claim 1 wherein the plasticizer is a naphthenic oil.

13. A hot melt pressure sensitive adhesive composition, wherein the hot melt pressure sensitive adhesive composition comprises:
a) from 12% by weight to 28% by weight of one or more styrenic block copolymer(s), wherein the styrenic block copolymer has a styrene content of from 10% by weight to 22% by weight and a di-block content of from 12% by weight to 65% by weight, and when more than one styrenic block copolymer is included, the styrenic block copolymers have a weight average styrene content of from 10% by weight to 22% by weight and a weight average di-block content of from 12% by weight to 65% by weight,
b) from 30% by weight to 65% by weight of one or more hydrogenated hydrocarbon tackifying agent(s), wherein the hydrogenated hydrocarbon tackifying agent has a weight average molecular weight (Mw) of at least 1000 grams/mole, and when more than one hydrogenated hydrocarbon tackifying agent is included, the hydrogenated hydrocarbon tackifying agents have a weight average of the weight average molecular weight (Mw) of at least 1000 grams/mole, and
c) from 10% by weight to 40% by weight of a plasticizer.

14. The hot melt pressure sensitive adhesive of claim 13 being free of non-hydrogenated C5 tackifying agents.

15. The hot melt pressure sensitive adhesive of claim 13 wherein the hydrogenated hydrocarbon tackifying agent comprises an aromatic modified hydrocarbon tackifying agent having an aromatic content of from 3% to 15% and an aromatic hydrocarbon tackifying agent having an aromatic content of from 40% to 100%.

## Patentansprüche

1. Absorbierender Artikel, umfassend:
a) eine Deckschicht;
b) eine Rückschicht;
c) optional einen Absorptionskern, der zwischen der Deckschicht und der Rückschicht angeordnet ist;
d) eine kleidungszugewandte Oberfläche; und
e) eine auf der kleidungszugewandten Oberfläche des absorbierenden Artikels angeordnete Schmelz-Haftklebstoffzusammensetzung, wobei die Schmelz-Haftklebstoffzusammensetzung umfasst:
i) 10 Gew.-% bis 35 Gew.-% eines oder mehrerer Styrol-Blockcopolymere, wobei das Styrol-Blockcopolymer einen Styrolgehalt von 10 Gew.-% bis 22 Gew.-% und einen Diblock-Anteil von 12 Gew.-% bis 65 Gew.-% aufweist und, wenn mehr als ein Styrol-Blockcopolymer enthalten ist, die Styrol-Blockcopolymere einen gewichtsmittleren Styrolgehalt von 10 Gew.-% bis 22 Gew.-% und einen gewichtsmittleren Diblock-Anteil von 12 Gew.-% bis 65 Gew.-% aufweisen,
ii) 30 Gew.-% bis 65 Gew.-% eines oder mehrerer hydrierter Kohlenwasserstoff-Klebrigmacher, wobei der hydrierte Kohlenwasserstoff-Klebrigmacher ein gewichtsmittleres Molekulargewicht Mw von mindestens 1000 g/mol aufweist und, wenn mehr als ein hydrierter Kohlenwasserstoff-Klebrigmacher enthalten ist, die hydrierten Kohlenwasserstoff-Klebrigmacher einen Gewichtsmittelwert des gewichtsmittleren Molekulargewichts Mw von mindestens 1000 g/mol aufweisen, und
iii) 10 Gew.-% bis 40 Gew.-% eines Weichmachers.

2. Absorbierender Artikel nach Anspruch 1, umfassend einen Absorptionskern.

3. Absorbierender Artikel nach Anspruch 1, wobei die Schmelz-Haftklebstoffzusammensetzung frei von Dioxinen ist.

4. Absorbierender Artikel nach Anspruch 1, wobei die Schmelz-Haftklebstoffzusammensetzung frei von nicht hydrierten C5-Klebrigmachern ist.

5. Absorbierender Artikel nach Anspruch 1, wobei der/die hydrierte(n) Kohlenwasserstoff-Klebrigmacher einen durchschnittlichen Ring-und-Kugel-Erweichungspunkt von 95 °C bis 120 °C aufweist/aufweisen.

6. Absorbierender Artikel nach Anspruch 1, wobei der/die hydrierte(n) Kohlenwasserstoff-Klebrigmacher einen durchschnittlichen Aromatengehalt von 10 Gew.-% bis 25 Gew.-% aufweist/aufweisen.

7. Absorbierender Artikel nach Anspruch 1, wobei das hydrierte Kohlenwasserstoffharz ausgewählt ist aus der Gruppe bestehend aus aromatisch modifiziertem Kohlenwasserstoffharz, aromatischem Kohlenwasserstoffharz und Kombinationen davon.

8. Absorbierender Artikel nach Anspruch 1, wobei das hydrierte Kohlenwasserstoffharz ein aromatisch modifiziertes Kohlenwasserstoffharz und ein aromatisches Kohlenwasserstoffharz umfasst.

9. Absorbierender Artikel nach Anspruch 1, wobei das Styrol-Blockcopolymer ausgewählt ist aus einer Gruppe bestehend aus Poly(styrol-b-isopren-b-styrol), Poly(styrol-b-(ethylen-co-butylen)-b-styrol) und Kombinationen davon.

10. Absorbierender Artikel nach Anspruch 1, wobei das/die Styrol-Blockcopolymer(e) eine gewichtsmittlere Schmelzflussrate gemäß ASTM D 1238 (200 °C, 5 kg) von 5 bis 40 g/10 min aufweist/aufweisen.

11. Absorbierender Artikel nach Anspruch 1, wobei das/die Styrol-Blockcopolymer(e) einen durchschnittlichen Diblock-Anteil von 20 Gew.-% bis 60 Gew.-% aufweist/aufweisen.

12. Absorbierender Artikel nach Anspruch 1, wobei der Weichmacher ein naphthenisches Öl ist.

13. Schmelz-Haftklebstoffzusammensetzung, wobei die Schmelz-Haftklebstoffzusammensetzung umfasst:
a) 12 Gew.-% bis 28 Gew.-% eines oder mehrerer Styrol-Blockcopolymere, wobei das Styrol-Blockcopolymer einen Styrolgehalt von 10 Gew.-% bis 22 Gew.-% und einen Diblock-Anteil von 12 Gew.-% bis 65 Gew.-% aufweist und, wenn mehr als ein Styrol-Blockcopolymer enthalten ist, die Styrol-Blockcopolymere einen gewichtsmittleren Styrolgehalt von 10 Gew.-% bis 22 Gew.-% und einen gewichtsmittleren Diblock-Anteil von 12 Gew.-% bis 65 Gew.-% aufweisen,
b) 30 Gew.-% bis 65 Gew.-% eines oder mehrerer hydrierter Kohlenwasserstoff-Klebrigmacher, wobei der hydrierte Kohlenwasserstoff-Klebrigmacher ein gewichtsmittleres Molekulargewicht Mw von mindestens 1000 g/mol aufweist und, wenn mehr als ein hydrierter Kohlenwasserstoff-Klebrigmacher enthalten ist, die hydrierten Kohlenwasserstoff-Klebrigmacher einen Gewichtsmittelwert des gewichtsmittleren Molekulargewichts Mw von mindestens 1000 g/mol aufweisen, und
c) 10 Gew.-% bis 40 Gew.-% eines Weichmachers.

14. Schmelz-Haftklebstoff nach Anspruch 13, der frei von nicht hydrierten C5-Klebrigmachern ist.

15. Schmelz-Haftklebstoff nach Anspruch 13, wobei der hydrierte Kohlenwasserstoff-Klebrigmacher einen aromatisch modifizierten Kohlenwasserstoff-Klebrigmacher mit einem Aromatengehalt von 3 % bis 15 % und einen aromatischen Kohlenwasserstoff-Klebrigmacher mit einem Aromatengehalt von 40 % bis 100 % umfasst.

## Revendications

1. Un article absorbant comprenant :
a) une couche supérieure ;
b) une couche inférieure ;
c) éventuellement un noyau absorbant disposé entre la couche supérieure et la couche inférieure ;
d) une surface orientée vers le vêtement ; et
e) une composition adhésive thermofusible sensible à la pression disposée sur la surface orientée vers le vêtement de l'article absorbant, la composition adhésive thermofusible sensible à la pression comprenant :
i) de 10 % en poids à 35 % en poids d'un ou plusieurs copolymère(s) séquencé(s) styrénique(s), dans lequel le copolymère séquencé styrénique a une teneur en styrène de 10 % en poids à 22 % en poids et une teneur en diblocs de 12 % en poids à 65 % en poids, et lorsque plus d'un copolymère séquencé styrénique est inclus, les copolymères séquencés styréniques ont une teneur moyenne en poids en styrène de 10 % en poids à 22 % en poids et une teneur moyenne en poids en diblocs de 12 % en poids à 65 % en poids,
ii) de 30 % en poids à 65 % en poids d'un ou plusieurs agent(s) tackifiant(s) hydrocarbonés hydrogénés, dans lequel l'agent tackifiant hydrocarboné hydrogéné a un poids moléculaire moyen en poids (Mw) d'au moins 1000 grammes/mole et, lorsque plus d'un agent tackifiant hydrocarboné hydrogéné est inclus, les agents tackifiants hydrocarbonés hydrogénés ont une moyenne pondérale du poids moléculaire moyen en poids (Mw) d'au moins 1000 grammes/mole, et
iii) de 10 % en poids à 40 % en poids d'un plastifiant.

2. L'article absorbant selon la revendication 1 comprenant un noyau absorbant.

3. L'article absorbant selon la revendication 1, dans lequel la composition adhésive thermofusible sensible à la pression est exempte de dioxines.

4. L'article absorbant selon la revendication 1, dans lequel la composition adhésive thermofusible sensible à la pression est exempte d'agents tackifiants C5 non hydrogénés.

5. L'article absorbant selon la revendication 1 dans lequel le ou les agents tackifiants hydrocarbonés hydrogénés a/ont un point de ramollissement moyen Bille et Anneau compris entre 95°C et 120°C.

6. L'article absorbant selon la revendication 1, dans lequel le ou les agents tackifiants hydrocarbonés hydrogénés possèdent une teneur aromatique moyenne de 10 % en poids à 25 % en poids.

7. L'article absorbant selon la revendication 1, dans lequel la résine hydrocarbonée hydrogénée est choisie dans le groupe constitué par une résine hydrocarbonée modifiée aromatique, une résine hydrocarbonée aromatique et leurs combinaisons.

8. L'article absorbant selon la revendication 1, dans lequel la résine hydrocarbonée hydrogénée comprend une résine hydrocarbonée modifiée aromatique et une résine hydrocarbonée aromatique.

9. L'article absorbant selon la revendication 1, dans lequel le copolymère séquencé styrénique est choisi parmi un groupe constitué de poly(styrène-b-isoprène-b-styrène), de poly(styrène-b-(éthylène-co-butylène)-b-styrène) et de leurs combinaisons.

10. L'article absorbant selon la revendication 1 dans lequel le ou les copolymères séquencés styréniques a/ont un indice de fluidité à chaud moyen en poids selon la norme ASTM D 1238 (200 °C, 5 kg) allant de 5 à 40 grammes/10 minutes.

11. L'article absorbant selon la revendication 1, dans lequel le ou les copolymères séquencés styréniques a/ont une teneur moyenne en diblocs de 20 % en poids à 60 % en poids.

12. L'article absorbant selon la revendication 1, dans lequel le plastifiant est une huile naphténique.

13. Une composition adhésive sensible à la pression thermofusible, dans laquelle la composition adhésive sensible à la pression thermofusible comprend :
a) de 12 % à 28 % en poids d'un ou plusieurs copolymère(s) séquencé(s) styrénique(s), dans lequel le copolymère séquencé styrénique a une teneur en styrène de 10 % à 22 % en poids et une teneur en diblocs de 12 % à 65 % en poids, et lorsque plus d'un copolymère séquencé styrénique est inclus, les copolymères séquencés styréniques ont une teneur en styrène moyenne en poids de 10 % à 22 % en poids et une teneur en diblocs moyenne en poids de 12 % à 65 % en poids,
b) de 30 % en poids à 65 % en poids d'un ou de plusieurs agent(s) tackifiant(s) hydrocarboné(s) hydrogéné(s), daas lequel l'agent tackifiant hydrocarboné hydrogéné a un poids moléculaire moyen en poids (Mw) d'au moins 1000 grammes/mole, et lorsque plus d'un agent tackifiant hydrocarboné hydrogéné est inclus, les agents tackifiants hydrocarbonés hydrogénés ont une moyenne pondérale du poids moléculaire moyen en poids (Mw) d'au moins 1000 grammes/mole, et
c) de 10 % en poids à 40 % en poids d'un plastifiant.

14. L'adhésif sensible à la pression thermofusible selon la revendication 13 est exempt d'agents tackifiants C5 non hydrogénés.

15. L'adhésif sensible à la pression thermofusible selon la revendication 13 dans lequel l'agent tackifiant hydrocarboné hydrogéné comprend un agent tackifiant hydrocarboné modifié aromatique ayant une teneur en aromatiques de 3 % à 15 % et un agent tackifiant hydrocarboné aromatique ayant une teneur en aromatiques de 40 % à 100 %.
